(19) **Europäisches Patentamt European Patent Office Office européen des brevets**

(11) **EP 4 173 617 A1**

(12) **EUROPEAN PATENT APPLICATION**

(43) Date of publication:
**03.05.2023  Bulletin 2023/18**

(21) Application number: **21382973.2**

(22) Date of filing: **28.10.2021**

(51) International Patent Classification (IPC):
***A61K 9/51*** (2006.01)

(52) Cooperative Patent Classification (CPC):
**A61K 9/0019; A61K 9/5146; A61K 33/26; A61K 47/50; A61P 31/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Consejo Superior De Investigaciones Científicas**
**28006 Madrid (ES)**

(72) Inventors:
- **BARBER CASTAÑO, Domingo F.**
  **28006 Madrid (ES)**
- **MORALES HERRERO, María del Puerto**
  **28006 Madrid (ES)**
- **LÓPEZ DE DIEGO, Marta**
  **28006 Madrid (ES)**
- **MULENS ARIAS, Vladimir**
  **28006 Madrid (ES)**

- **PORTILLA TUNDIDOR, Yadileiny**
  **28006 Madrid (ES)**
- **DAVIU BOU, Neus**
  **28006 Madrid (ES)**
- **VEINTEMILLAS VERDAGUER, Sabino**
  **28006 Madrid (ES)**
- **GALLO CÓRDOVA, Alvaro**
  **28006 Madrid (ES)**
- **VILLAMAYOR CORONADO, Laura**
  **28006 Madrid (ES)**
- **LÓPEZ GARCÍA, Darío**
  **28006 Madrid (ES)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

(54) **IRON OXIDE NANOPARTICLES WITH ANTIVIRAL ACTIVITY AGAINST SARS AND MERS CORONAVIRUS**

(57)  *Coronaviridae* viruses usually cause mild respiratory disease in humans, nevertheless a new infectious disease caused by *Severe acute respiratory syndrome coronavirus* 2 (SARS-CoV-2) has spread globally since December 2019, resulting in the ongoing 2019-2021 coronavirus pandemic. The present invention discloses the use of coated iron oxide nanoparticles (IONPs) for use in the treatment and/or prevention of viral infections caused by *Coronaviridae,* especially those caused by respiratory syndrome-related coronaviruses such as SARS-CoV, SARS-CoV-2 and MERS-CoV. The iron oxide nanoparticles are coated with biocompatible molecules and polymers which bind to the nanoparticle core via oxygen, nitrogen and sulfur donor atoms (e.g. dextran, (3-aminopropyl)triethoxysilane or dimercaptosuccinic acid, respectively). The IONPs were found stable, non-cytotoxic *in vitro* and can efficiently impair virus replication, transcription and production of infectious virus. Furthermore, the IONPs are suitable for oral, intranasal or parenteral administration in combination with a pharmaceutical carrier.

EP 4 173 617 A1

**Description**

**FIELD OF THE INVENTION**

The present invention relates to coated iron oxide nanoparticles for use in the treatment and/or prevention of viral infections caused by *Coronaviridae,* especially those caused by respiratory syndrome-related coronaviruses selected from the species *"Severe acute respiratory syndrome-related coronavirus"* (such as SARS-CoV and SARS-CoV-2) and *"Middle East respiratory syndrome-related coronavirus"* (MERS).

**BACKGROUND**

Viruses are accountable for the common cold, influenza, AIDS, SARS, MERS, COVID-19, chikungunya, Dengue fever, zika fever, yellow fever, West Nile fever, hepatitis B, hepatitis C, hepatitis E, Ebola virus disease and rabies, among other notable human diseases. The viruses of the family *Coronaviridae* are enveloped viruses with a positive-sense single-stranded RNA genome and a nucleocapsid of helical symmetry. The name of the family is derived from the Latin corona, meaning "crown" or "halo", which refers to the characteristic appearance reminiscent of a solar corona around the virions (virus particles) when viewed under two-dimensional transmission electron microscopy, due to the surface being covered in club-shaped protein spikes.

Several members of the family *Coronaviridae* usually cause mild respiratory disease in humans (Corman et al., 2019). However, other family members such as the severe acute respiratory syndrome coronavirus (SARS-CoV) and the Middle East respiratory syndrome coronavirus (MERS-CoV) are transmitted from animals to humans and cause severe respiratory diseases in afflicted individuals, SARS and MERS, respectively (Fehr et al., 2017).

In December 2019 a new infectious disease caused by severe acute respiratory syndrome coronavirus 2 (SARS-CoV-2) was first identified in Wuhan, the capital of China's Hubei province, and has since spread globally, resulting in the ongoing 2019-2021 coronavirus pandemic. The disease has been given the name Coronavirus disease 2019 or COVID-19. COVID-19 has evolved into an appalling global pandemic worldwide.

The etiological agent of this severe respiratory syndrome was named SARS-CoV-2 due to its genetic similarity to the Severe Acute Respiratory Syndrome coronavirus (SARS-CoV) that caused the 2003 epidemic outbreak in different areas of Southeast Asia.

SARS-CoV-2 can replicate efficiently in asymptomatic patients, facilitating the spread of the virus worldwide, given that high replication levels are not necessarily associated with clinical manifestations of the disease that may restrict infected patient mobility. While silent or causing mild symptoms in a majority of cases, SARS-CoV-2 may lead to COVID-19, which is primarily but not exclusively characterized by severe pneumonia that may lead to death in a fraction of the population, most notably the elderly.

Common symptoms include fever and cough and loss of taste or smell, which may be accompanied by other such as muscle pain, diarrhoea or sore throat. While the majority of cases result in these mild symptoms, some patients progress to hypoxia, bilateral pneumonia and multi-organ failure.

COVID-19 can seriously damage the respiratory system, with some patients developing acute respiratory infection symptoms, and even acute respiratory distress syndrome (ARDS), acute respiratory failure and other complications, such as in the cardiovascular system (T. J. Guzik et al, Cardiovasc Res 2020, 116(10), 1666-1687) and central nervous system (C. Iadecola et al, Cell 2020, 183(1), 16-27) which in some cases even lead to death.

Numerous efforts are being put into the development of preventive vaccines and while this is certainly the best approach to contain future outbreaks, it is clear that additional strategies need to be in place for patients that already acquired the infection.

A timely discovery of new drugs is not a viable strategy to contrast a pressing pandemic global situation, therefore the repurposing of existing drugs with established safety profile to treat COVID-19 seems more effective.

Metal and metal oxides nanoparticles have shown antiviral properties against a wide range of viruses such as influenza, HIV-1, HBV, etc.

Iron oxide nanoparticles (IONPs) have considerable potential to be used as nanomedicines for different biomedical applications, including targeted drug release or magnetic resonance imaging. Previous studies have shown that the accumulation of IONPs inside cells induces the production of reactive oxygen species (ROS), as a consequence of IONPs degradation inside lysosomes, which trigger oxidative stress, that in turn induces the transcriptional regulation of oxidative stress-inducible genes (Portilla et al. ACS Appl. Mater. Interfaces 2021, 13(7):7924-7944). The induction of oxidative stress by IONPs might be used therapeutically in the treatment of viral infections.

In a recent report, the *in vitro* antiviral activity of IONPs against H1N1 influenza infection was analyzed. Results from plaque inhibition assays and quantitative real-time PCR (qPCR) of viral transcripts in the presence of IONP suggested antiviral activity of IONPs against influenza (Kumar et al. J. Infect. Chemother. 2019, 25(5):325-329). Another report suggested that, as a consequence of their ability to induce the production of reactive oxygen species, IONPs can catalyze lipid peroxidation of the viral lipid envelope and reduce the infectivity of H1N1, H5N1, and H7N9 subtype viruses (Kim et al. Small 2017, 13(32): 1700818). In addition, it has been shown that the treatment of cells with ferric ammonium citrate (FAC) reduces influenza virus replication and production (Wang et al. Cell Discovery. 2018, 4:14). However, despite being iron oxide nanoparticles known in therapeutics there have not been efforts to test them in the treatment of severe respiratory syndrome-related coronavirus.

A theoretical study (Y. Abo-Zeid et al., Eur. J. Pharm. Sci., 2020, 153, 105465) has shown that discrete $Fe_2O_3$ and $Fe_3O_4$ molecules can interact with the S1-RBD protein receptor domain, which is used by SARS-CoV-2 for its internalization into the host cell and subsequent replication cycling, however this modelling study does not address aspects of crucial importance such as the size or the coating of the nanoparticle. These parameters not only determine the therapeutic efficacy against the biological target but may also have an influence on the resulting toxicity of the nanoparticles. Indeed, inorganic nanoparticles have been of limited use in clinical practice due to toxicity derived from size, shape, charge and surface chemistry. For example, gold nanoparticles have been found toxic at a size of 1.4 nm but not at higher particle size (Y. Pan, Small, 2007, 3, 1941-1949). Therefore, specifically tailored metal and metal oxide nanoparticles are needed to solve the limitations of current methods of treatments of *Coronaviridae* infections.

## BRIEF DESCRIPTION OF THE INVENTION

With the start of the COVID-19 pandemic, the inventors have found that iron oxide nanoparticles (IONPs) display antiviral activity against SARS-CoV-2. The IONPs can be synthetized or, as an alternative, are commercially available and already approved by regulatory agencies as contrast agents (e.g. FeraSpin R, nanoPET Pharma GmbH). Since the latter are authorized and regulated for medical applications, they could be easily repositioned as antiviral drugs against SARS and MERS Coronaviruses. Furthermore, it has been described that the severity of COVID-19 was negatively correlated with serum iron levels (Zhao et al. Open Forum Infectious Diseases. 2020, 7(7):ofaa250; Sonnweber et al. Respiratory Research. W2020, 21(1):276), therefore, in addition to the likely direct antiviral activity of IONPs, the treatment with IONPs could improve patient outcomes. In addition to that, by coating the nanoparticles with a suitable organic compound, advantageous properties are displayed. Firstly, the coating confers biocompatibility to the nanoparticle which can therefore be used at a therapeutically effective concentration in living cells without causing evident cytotoxicity. Furthermore, the biocompatible coating surrounding the iron plays crucial roles in stabilizing the iron core, slowing down the release of iron, protecting the particles from further aggregation, as well as sustaining the particles in a colloidal suspension that can be intravenously injected.

Thus, a first aspect of the invention provides coated iron oxide nanoparticles for use in the treatment or prevention of an infection caused by respiratory syndrome-related coronaviruses selected from the species *"Severe acute respiratory syndrome-related coronavirus"* and *"Middle East respiratory syndrome-related coronavirus"*.

A second aspect of the invention relates to a pharmaceutical composition for use in the treatment or prevention of an infection by respiratory syndrome-related coronaviruses selected from the species *"Severe acute respiratory syndrome-related coronavirus"* and *"Middle East respiratory syndrome-related coronavirus"*, said composition comprising as active ingredient an effective amount of coated iron oxide nanoparticles.

## BRIEF DESCRIPTION OF THE DRAWINGS

Figure 1 shows TEM images of the nanoparticles used in the invention.

Figure 2 shows the magnetic and X-ray characterization of the nanoparticles used in the invention.

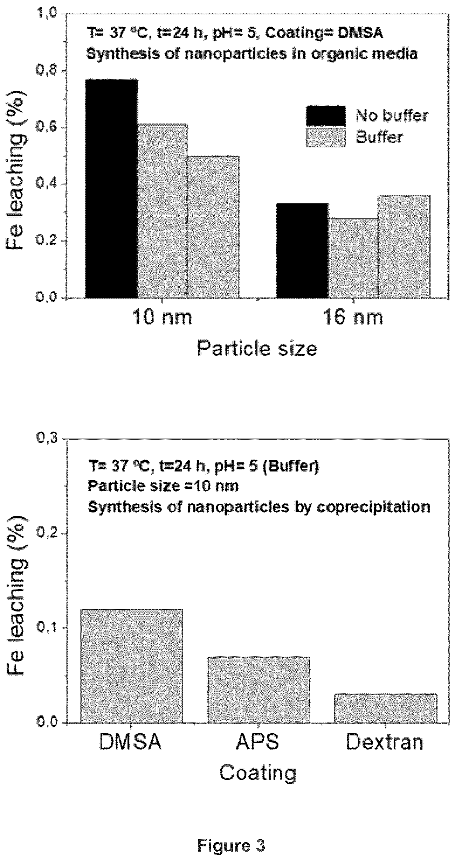

Figure 3 shows data related to iron leaching from the iron oxide nanoparticles under the specified conditions depending on the size of the DMSA-coated nanoparticles (top) and on the type of coating (bottom).

Figure 4 shows the IONPs cytotoxicity in Vero E6 cell lines as determined by PrestoBlue assay. The nanoparticle cytotoxicity was determined at 24 h with different doses of FeraSpin R, DMSA-IONP-10, APS-IONP-10 and DMSA-IONP-16 (56 $\mu$g Fe/mL = 1 mM).

Figure 5 shows the time-dependent IONPs uptake by Vero E6 cells (56 $\mu$g Fe/mL = 1 mM). The internalization of APS-IONP-10 (a), FeraSpin R (b), DMSA-IONP-10 (c) and DMSA-IONP-16 (d) by Vero E6 cells was measured at 3, 6 and 24 h.

Figure 6 shows the effect of therapeutic and prophylactic treatment with IONPs on SARS-CoV-2 titers in Vero E6-infected cells (1 mM = 56 $\mu$g/mL).

Figure 7 shows the effect of therapeutic and prophylactic treatments with IONPs on SARS-CoV-2 replication (by quantification of genomic RNA, gRNA, by RT-qPCR) and transcription (by quantification of subgenomic RNA, sgRNA, by RT-qPCR) in Vero E6-infected cells (56 $\mu$g Fe/mL = 1 mM).

## DETAILED DESCRIPTION OF THE INVENTION

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention.

Unless defined otherwise, all technical and scientific terms used herein have the same meaning as commonly understood to one of ordinary skill in the art to which this disclosure belongs. As used herein, the singular forms "a", "an" and "the" include plural reference unless the context clearly dictates otherwise.

The present invention is based on the finding that administration of coated iron oxide nanoparticles can be used for the treatment or prevention of *"Severe acute respiratory syndrome-related coronavirus"* and *"Middle East respiratory syndrome-related coronavirus"*. Specifically, it was found that the administration of coated iron oxide nanoparticles in cell cultures infected by SARS-CoV-2 produced prophylactic and therapeutic effects.

The iron oxide nanoparticles used in the present invention are surface-modified nanoparticles coated with organic molecules such as biocompatible organic molecules, surfactants, polymers and biomacromolecules. The biocompatible coating surrounding the iron oxide plays crucial roles in stabilizing the iron oxide core, slowing down the release of iron, protecting the particles from further aggregation, as well as sustaining the particles in a colloidal suspension that can be intravenously injected. The coating may partially or completely cover the surface of the iron oxide core to which it is bound. When the coating completely covers the iron oxide core, a homogeneous monolayer of the biocompatible molecule is surrounding the spherical or quasi-spherical iron oxide core. Alternatively, more than one layer can cover the iron oxide particle core by hydrophobic and/or hydrophilic interactions between the organic molecules of the coating.

As used herein, the term "iron oxide" is used to denote a chemical compound composed of iron and oxygen. The iron oxide may be of any known magnetism, including, magnetic iron oxide, ferromagnetic iron oxide, ferrimagnetic iron oxide, and anti-ferromagnetic iron oxide, wherein the iron may be composed of one or both of ferric iron ($Fe^{3+}$) and ferrous iron ($Fe^{2+}$). Non-limiting examples of iron oxides include FeO (wüstite), $Fe_3O_4$ (magnetite, $Fe^{II}Fe^{III}_2O_4$) and $Fe_2O_3$ having different forms such as, $\alpha$-$Fe_2O_3$ (hematite), $\beta$-$Fe_2O_3$, $\gamma$-$Fe_2O_3$ (maghemite) and $\epsilon$-$Fe_2O_3$.

As used herein "iron oxide nanoparticles" (or "IONPs") are iron oxide nanoparticles having a mean size in the nanometer scale, typically ranging between 1 nm to 500, preferably from 1 nm to 200 nm, and more preferably between 1 nm to 150 nm or even more preferably from 1 nm to 100 nm. In the context of the invention, such nanoparticles are superparamagnetic iron oxide nanoparticles that are coated with inorganic materials such as silica, carbon, non-metal elements, metal oxides and metal sulfides or organic molecules such as biocompatible organic molecules, surfactants, polymers and biomacromolecules. Preferably, the superparamagnetic iron oxide nanoparticles are coated with biocompatible organic molecules, surfactants, polymers and biomacromolecules,

In preferred embodiments, the nanoparticles are superparamagnetic iron oxide nanoparticles (so-called SPIONs) which comprise a core comprising a mixture of magnetite and maghemite. Superparamagnetic iron oxide nanoparticles (SPIONs) possess desirable properties such as controllable size, large surface area-to-volume ratio, and non-toxicity. The application of an external magnetic field can guide coated SPIONs to the desired site allowing a direct and specific therapeutic effect with minimum side effects. The superparamagnetic iron oxide nanoparticles have been characterized in terms of their intrinsic magnetic properties. In particular, the coated superparamagnetic iron oxide nanoparticles display saturation magnetization (Ms) values between 40 and 140 A m$^2$/kg$_{Fe}$. The saturation magnetization (Ms) is the maximum magnetic moment per unit volume or mass for a magnetic material.

In a particular embodiment, the size of the coated iron oxide nanoparticle is comprised between 2 and 50 nm. In a preferred embodiment, the size of the coated iron oxide nanoparticle is comprised between 4 and 20 nm.

The particle size can be determined by different techniques that are known in the art such as transmission electron microscopy (TEM) or, if a crystalline phase is present, X-ray diffraction (XRD). The values of particle size obtained by the techniques known in the art are in agreement within the experimental error.

In the present invention, the particle size is determined by transmission electron microscopy (TEM).

In another particular embodiment, the hydrodynamic size of the coated iron oxide nanoparticle is comprised between 25 and 100 nm, preferably between 35 and 75 nm. In contrast to the particle size, the hydrodynamic size (alternatively called "hydrodynamic diameter" throughout the description) relates to the behaviour of the nanoparticle in a fluid and it represents the diameter of a hard sphere that diffuses at the same speed as the nanoparticle being measured. The hydrodynamic diameter of the coated iron oxide nanoparticle will depend on the size of the iron oxide core with no coating, but also on the type of coating, as well as on the type and concentration of any ions in the medium. The hydrodynamic size can be calculated by photon correlation spectroscopy.

In a particular embodiment, the coating of the iron oxide nanoparticle is a neutrally charged organic molecule selected from the group consisting of biocompatible organic molecules, surfactants, polymers and biomacromolecules.

A "biocompatible organic molecule" is defined as an organic compound with low molecular weight, usually up to 1000 Dalton and size on the order of 1 nm that can display a biological function by binding to a biological macromolecule to alter its native activity or function. Organic biocompatible molecules may include without being limited thereto, amino acids, mono- or oligo-saccharides, citric acids, succinic acids, mercapto carboxylic acids, carboxylic acids, amines, silanes, vitamins, cyclodextrins.

A "surfactant" is defined as an organic compound that is amphiphilic, namely it contains both hydrophobic and hydrophilic groups. Example of surfactants include long fatty carbon chains with a hydrophilic head such as fatty acids, polyols, petroleum sulphonate, alkylbenzenesulphonates, naphthalene sulphonates, olefin sulphonates, alkyl sulphates, carboxylates, sulphonates, sulphated natural oils, sulphated esters, sulphated alkanolamides, ethoxylated and sulphated alkylphenols (linear or branched), ethoxylated aliphatic alcohols, polyoxyethylene surfactants, carboxylic esters, polyethylene glycol esters, anhydrosorbitol ester, glycol esters of fatty acids (oleic acid and lauric acid), carboxylic amides, monoalkanolamine condensates, polyoxyethylene fatty acid amides, quaternary ammonium salts, alkyl phosphonates such as dodecyl phosphonate, hexadecyl phosphonate, dihexadecyl phosphonate.

Polymers may be selected for example from natural polymers such as for example dextran, starch, gelatin, chitosan; or from synthetic polymers such as for example polyethylene glycol (PEG), polyvinyl alcohol (PVA), polylactide acid (PLA), alginate, polyacrylic acid (PAA), polymethylmethacrylate (PMMA).

Biomacromolecules that can be used for coating the iron oxide nanoparticles include, for example, proteins, polypeptides, sugars and antibodies.

In one embodiment, the neutrally charged organic molecule used for coating the iron nanoparticle contains -XH groups, where X is a heteroatom selected from O, S and N. In another embodiment, the neutrally charged organic molecule used for coating the iron nanoparticle contains simultaneously different -XH group, wherein X is independently selected from O, S and N.

In a preferred embodiment, the neutrally charged organic molecule containing -XH groups as defined above is a sugar, a thiol-containing sugar, a mercaptan or an amine. The presence of hydroxyl, thiol groups and/or amino groups, particularly those in sugars/thiol-containing sugars and mercaptans, has a beneficial effect to the stability of iron oxide nanoparticles with a narrow size range since such ligands bind strongly to the nanoparticle surface avoiding particle aggregation and selectively blocking metal sites that might otherwise give rise to side reactions. Examples of preferred neutrally charged organic molecules containing -XH groups include citric acids, cyclodextrins, polysaccharides, polyethyleneglycol, dextran, carboxydextran, alginate (e.g. sodium alginate, calcium alginate, alginate sulfate), mercaptans, mercapto carboxylic acids, alkylamines.

In one embodiment, the neutrally charged organic molecule used for coating the iron oxide nanoparticle has a molecular weight between 80 and 100000 Dalton, preferably between 80 and 50000 Dalton.

In a preferred embodiment, the iron oxide nanoparticle is coated with dextran, carboxydextran, carboxymethyldextran, starch, PEGylated starch, (3-aminopropyl)triethoxysilane or dimercaptosuccinic acid (DMSA).

In one embodiment, the iron oxide nanoparticles comprise an iron oxide core covered with carboxydextran. Particularly, the resulting coated iron oxide nanoparticle has a particle size between 2 and 15 nm, preferably between 4 and 10 nm, and even more preferably the particle size is of about 5 nm. In a more particular embodiment, the hydrodynamic size is of between 50 and 80 nm. In a more particular embodiment, the saturation magnetization is comprised between 80 and 120 A $m^2/kg_{Fe}$.

In a particular embodiment, the iron oxide nanoparticles coated with carboxydextran are commercially available under the trademark FeraSpin™ R (which are approved for use as diagnostic imaging agent for *in vivo* MRI in mice).

In a particular embodiment, the iron oxide nanoparticles coated with carboxydextran are commercially available under the trademark Resovist® (which are approved for use as diagnostic imaging agent for small liver lesions).

In a particular embodiment, the iron oxide nanoparticles coated with carboxydextran are commercially available under the trademark Magtrace®, which are approved for use as tracer for Sentinel Lymph Node Biopsy (SLNB).

Other commercially available formulations that have been approved by regulatory agencies consist of coated iron oxide nanoparticles and could be potentially effective for the therapeutic and prophylactic treatment of SARS and MERS coronavirus. Among these formulations, Endorem® (Europe), also branded as Feridex IV® in USA, and Sinerem® (Europe), also branded as Combidex® in USA, are superparamagnetic iron oxide nanoparticles coated with dextran that are used to detect metastatic disease in liver/spleen and lymph nodes, respectively, and are administered intravenously. The size of the nanoparticles is comprised between 4-6 nm and 4-15 nm for Endorem® and Sinerem®, respectively. The hydrodynamic size of Endorem® nanoparticles is in the range 80-150 nm, while Sinerem® nanoparticles have a considerably smaller hydrodynamic size (15-30 nm).

Ferumoxytol® comprises superparamagnetic iron oxide nanoparticles coated with carboxymethyl-dextran, with a particle size of 3-7 nm and a hydrodynamic size of about 30 nm, and is administered intravenously for the MRI of the gastrointestinal lumen.

Clariscan® is used as a MR angiography agent (by intravenous administration) and consist of superparamagnetic iron oxide nanoparticles particles coated with a carbohydrate functionalized with polyethylene glycol (PEG) coating with a 4 to 7 nm particle size and hydrodynamic size of about 20 nm.

In particular embodiment, the iron oxide nanoparticles comprise an iron oxide core covered with dextran. In particular embodiment, the iron oxide nanoparticles coated with dextran have particle size between 2 and 20 nm, preferably between 4 and 16 nm. In another particular embodiment, the iron oxide nanoparticles coated with dextran are commercially available under the trademark Endorem®. In another particular embodiment, the iron oxide nanoparticles coated with dextran are commercially available under the trademark Sinerem®.

In particular embodiment, the iron oxide nanoparticles comprise an iron oxide core covered with carboxymethyl-dextran. In particular embodiment, the iron oxide nanoparticles coated with carboxymethyl-dextran have particle size between 2 and 10 nm, preferably between 3 and 7 nm. In another particular embodiment, the iron oxide nanoparticles coated with carboxymethyl-dextran are commercially available under the trademark Ferumoxytol®.

In particular embodiment, the iron oxide nanoparticles comprise an iron oxide core covered with PEGylated starch. In particular embodiment, the iron oxide nanoparticles coated with PEGylated starch have particle size between 2 and 10 nm, preferably between 4 and 7 nm. In another particular embodiment, the iron oxide nanoparticles coated with PEGylated starch are commercially available under the trademark Clariscan®. A PEGylated starch coating is obtained from the derivatization of starch with poly(ethylene glycol) (PEG), a non-volatile plasticizer typically used for starch gelatinization, of at least an hydroxyl group along the chain of glucose monomers.

In another embodiment, the iron oxide nanoparticles comprise an iron oxide core covered with dimercaptosuccinic acid. Particularly, the resulting coated iron oxide nanoparticle has a particle size between 4 and 20 nm. In a more particular embodiment, the hydrodynamic size is of between 50 and 80 nm. In a more particular embodiment, the saturation magnetization is comprised between 50 and 120 A $m^2/Kg_{Fe}$.

NanoTherm® AS1 is an aqueous dispersion of superparamagnetic iron oxide nanoparticles in the form of magnetite ($Fe_3O_4$) with a mean diameter of 12 nm coated with (3-aminopropyl)triethoxysilane. The fluid, developed by MagForce Nanotechnologies AG, is approved by EMA for use in brain tumors since 2010. In another embodiment, the iron oxide nanoparticles comprise an iron oxide core covered with (3-aminopropyl)triethoxysilane. Particularly, the resulting coated iron oxide nanoparticle has a particle size between 5 and 15 nm, preferably between 7 and 12 nm, and even more preferably the particle size is about 9 nm. In a more particular embodiment, the hydrodynamic size is of between 60 and 125 nm. In a more particular embodiment, the saturation magnetization is comprised between 50 and 120 A $m^2/kg_{Fe}$.

In another particular embodiment, the iron oxide nanoparticles coated with (3-aminopropyl)triethoxysilane are commercially available under the trademark NanoTherm® AS1.

Uses of the iron oxide nanoparticles

The invention relates to coated iron oxide nanoparticles as described above for use in the treatment or prevention of viral infections by RNA-viruses, preferably of the family *Coronaviridae,* especially caused by the respiratory syndrome-related coronaviruses selected from the species *"Severe acute respiratory syndrome-related coronavirus"* (such as SARS-CoV-2 and SARS-CoV) and *"Middle East respiratory syndrome-related coronavirus"* (MERS-CoV).

The invention also relates to a method of treating or preventing viral infections by RNA-viruses, preferably of the family *Coronaviridae,* especially caused by the respiratory syndrome-related coronaviruses selected from the species *"Severe acute respiratory syndrome-related coronavirus"* (such as SARS-CoV-2 and SARS-CoV) and *"Middle East respiratory syndrome-related coronavirus"* (MERS-CoV) in a subject, said method comprising administering to said subject a therapeutically effective amount of coated iron oxide nanoparticles.

The term "viruses of the family *Coronaviridae",* as used herein, is used to designate any viral species of the taxonomic family *"Coronaviridae"* including those of the genera *"Alphacoronavirus", "Betacoronavirus",* "*Gammacoronavirus*" and *"Deltacoronavirus".*

In a preferred embodiment of the present invention, the viruses are selected from betacoronaviruses, in particular from the lineage "*Sarbecovirus*" and still more preferably from the species *"Severe acute respiratory syndrome-related coronavirus"* (such as SARS-CoV and SARS-CoV-2) and *"Middle East respiratory syndrome-related coronavirus"* (MERS-CoV).

In a more preferred embodiment, the viral infection is an infection caused by the species *"Severe acute respiratory syndrome-related coronavirus".* In a more particular embodiment, the viral infection is an infection by SARS-CoV-2 virus.

The terms "treating", "treatment", and "therapeutic treatment", as used herein, means reversing, alleviating, inhibiting the progress of, the disease or condition to which such term applies, or one or more symptoms of such disease or condition, such as lowering the viral load in a patient with respect to pre-treatment levels.

The terms "preventing", "prevention", and "prophylactic treatment" as used herein, means avoiding or inhibiting the onset of one or more symptoms of coronavirus infections such as fever, cough, shortness of breath, muscle pain, sputum production, diarrhoea, sore throat, loss of smell, pneumonia and abdominal pain.

The terms "SARS-CoV-2 titers" or "viral titers" as used herein, refers to the concentration of infectious viral particles as measured in the extracellular media thus a reduction of SARS-CoV-2 titers means a reduction of the concentration of infectious SARS-CoV-2 viral particles compared to a control in untreated cells.

Preferably, the IONPs disclosed herein, are used for the treatment and/or prevention of viral infections by *"Severe acute respiratory syndrome-related coronavirus"* and most preferably for the treatment and/or prevention of viral infections by SARS-CoV-2.

In another particular embodiment, the viral infection is an infection by *"Severe acute respiratory syndrome-related coronavirus"* and most preferably by SARS-CoV-2.

The treatment of cells infected by SARS-CoV-2 with IONPs reduced viral titers, when cells were treated with IONPs before (prophylactic treatment) or after (therapeutic treatment) SARS-CoV-2 infection. In preferred embodiments, the prophylactic treatment of cells with IONPs reduces SARS-CoV-2 titers by at least 90%, preferably by 95%.

In one embodiment, the prophylactic treatment of cells infected by SARS-CoV-2 with iron oxide nanoparticles covered by dimercaptosuccinic acid reduces SARS-CoV-2 titers by at least 70%, preferably by at least 90%.

In one embodiment, the therapeutic treatment of cells infected by SARS-CoV-2 with iron oxide nanoparticles covered by dimercaptosuccinic acid reduces SARS-CoV-2 titers by at least 70%, preferably by at least 90%.

In one embodiment, the prophylactic treatment of cells infected by SARS-CoV-2 with FeraSpin™ R nanoparticles reduces SARS-CoV-2 titers by at least 70%.

In one embodiment, the therapeutic treatment of cells infected by SARS-CoV-2 with FeraSpin™ R nanoparticles reduces SARS-CoV-2 titers by at least 60%.

Additionally, the treatment of cells infected by SARS-CoV-2 with IONPs directly impaired SARS-CoV-2 virus replication and/or transcription, when cells were treated with IONPs before (prophylactic treatment) or after (therapeutic treatment) SARS-CoV-2 infection. The amount of genomic RNA (gRNA), which determines virus replication, and gene 7 subgenomic messenger RNA (sg mRNA), which determines virus transcription, can be determined by Real-Time Quantitative Reverse Transcription PCR (qRT-PCR). In some embodiments, the treatment of cells before or after SARS-CoV-2 infection with coated iron oxide nanoparticles decreases the amounts of gRNA and gene 7 sg mRNA in both therapeutic and prophylactic treatments.

In a particular embodiment, the prophylactic treatment of cells infected by SARS-CoV-2 with iron oxide nanoparticles covered by dimercaptosuccinic acid reduces the amount of viral gRNA by at least 90%, preferably by at least 95%.

In a particular embodiment, the prophylactic treatment of cells infected by SARS-CoV-2 with iron oxide nanoparticles covered by dimercaptosuccinic acid reduces the amount of viral gene 7 sg mRNA by at least 90%, preferably by at least 95%.

In a particular embodiment, the therapeutic treatment of cells infected by SARS-CoV-2 with iron oxide nanoparticles covered by dimercaptosuccinic acid reduces the amount of viral gRNA by at least 90%, preferably by at least 95%.

In a particular embodiment, the therapeutic treatment of cells infected by SARS-CoV-2 with iron oxide nanoparticles covered by dimercaptosuccinic acid reduces the amount of viral gene 7 sg mRNA by at least 90%, preferably by at least 95%.

In a particular embodiment, the prophylactic treatment of cells infected by SARS-CoV-2 with FeraSpin™ R nanoparticles reduces the amount of viral gRNA by at least 60%, preferably by at least 70%.

In a particular embodiment, the prophylactic treatment of cells infected by SARS-CoV-2 with FeraSpin™ R nanoparticles reduces the amount of viral gene 7 sg mRNA by at least 80%.

In a particular embodiment, the therapeutic treatment of cells infected by SARS-CoV-2 with FeraSpin™ R nanoparticles reduces the amount of viral gRNA by at least 80%

In a particular embodiment, the therapeutic treatment of cells infected by SARS-CoV-2 with FeraSpin™ R nanoparticles reduces the amount of viral gene 7 sg mRNA by at least 80%

The nanoparticles for use according to the invention may be administered by any appropriate route (via), such as, oral (e.g., oral, sublingual, etc.) or parenteral (e.g., subcutaneous, intramuscular, intravenous, etc.). The nanoparticles for use according to the invention may also be administered intranasally, preferably in the form of an aerosol.

In particular, the nanoparticles for use according to the invention are administered as a pharmaceutical composition, which comprises the coated iron oxide nanoparticles and one or more pharmaceutically acceptable excipients.

The coated iron oxide nanoparticles can be used in a pharmaceutically acceptable composition that comprises the coated nanoparticles embedded within a carrier, such as hydrogels, liposomes or shell coating. Other typical carriers are known to the person skilled in the art.

In another embodiment, the pharmaceutical composition comprising the coated nanoparticles embedded within a carrier, such as hydrogels, liposomes or shell coating further comprises one or more pharmaceutically acceptable excipients.

In an embodiment, the nanoparticles for use according to the invention are administered in combination with one or more pharmaceutically acceptable excipients via oral or parenteral administration.

In a preferred embodiment, the coated iron oxide nanoparticles for use according to the invention are administered in combination with one or more pharmaceutically acceptable excipients by intravenous (i.v), intracoronary (i.c), intramuscular (i.m), intraperitoneal (i.p), or subcutaneous (s.c) injection.

In a particular embodiment, the coated iron oxide nanoparticles for use according to the invention are administered intranasally, preferably in the form of an aerosol.

The term "pharmaceutically acceptable excipient" refers to an inert, non-toxic vehicle, diluent, or adjuvant is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are known by the skilled person. The pharmaceutically acceptable excipient necessary to manufacture the desired pharmaceutical composition of the invention will depend, among other factors, on the elected administration route. Said pharmaceutical compositions may be manufactured according to conventional methods known by the skilled person in the art.

The compounds for use according to the invention may be administered in a "therapeutically effective amount", i.e. a non-toxic but sufficient amount of the corresponding compound to provide the desired effect. The amount that is "effective" will vary from subject to subject, depending on the age and general condition of the individual, the particular compound administered, and the like. However, an appropriate amount in any individual case may be determined by one of ordinary skill in the art using routine experimentation.

The iron oxide nanoparticles are administered and dosed taking into account the clinical condition of the individual, the site and method of administration, scheduling of administration, patient age, sex, body weight and other factors known to medical practitioners. The nanoparticles for use according to the invention will typically be administered once or more times a day, for example 1, 2, 3 or 4 times daily over a period of several days, with typical total daily doses depending on the particular compound and severity of the disease, and may be easily determined by the skilled practitioner.

By way of example, typical total doses for the nanoparticles of the invention are in the range of 0.1-25 mg/Kg body weight, preferably in the range of 1-10 mg/Kg body weight, even more preferably in the range of 1.5-5 mg/Kg body weight, when administered as single dose or up to 5 consecutive doses on alternate days.

The pharmaceutical compositions may be prepared using standard methods such as those described or referred to in the Spanish, European and US Pharmacopoeias and similar reference texts.

The term "subject" refers to a mammal, e.g., a human.

The nanoparticles for use according to the invention may be administered as the sole active ingredient or in combination with other active ingredients. In a particular embodiment, the nanoparticles are used as the sole active ingredient. In another particular embodiment, the nanoparticles are used in combination with other active ingredients such as one or more anti-inflammatory agents. Examples of anti-inflammatory agents which may be combined with the iron oxide particles include, without being limited thereto, steroidal anti-inflammatory drugs and non-steroidal anti-inflammatory drugs (NSATD). The additional agent may be administered to the subject before, concomitant or after administration of the iron oxide nanoparticles. When administered together with the iron oxide particles, the two (or more) may be in the same formulation or formulated in two different formulations.

In a particular embodiment, the nanoparticles of the invention are used in combination with one or more compounds useful in the treatment of viral infections by RNA-viruses, preferably of the family *Coronaviridae,* especially by the respiratory syndrome-related coronaviruses selected from the species *"Severe acute respiratory syndrome-related coronavirus"* (such as SARS-CoV-2 and SARS-CoV) and *"Middle East respiratory syndrome-related coronavirus"* (MERS-CoV). Some compounds that have been disclosed as potentially useful are selected from the group consisting of clofazimine, indomethacine, invermectin, disulfiram, boceprevir, paritaprevir, telaprevir, simeprevir, remdesivir, ribavirin, favipiravir, chloroquine, hydroxychloroquine, lopinavir, ritonavir, umifenovir and recombinant type I interferon, preferably clofazimine, indomethacine, invermectin, disulfiram, boceprevir, paritaprevir, telaprevir, simeprevir, remdesivir, lopinavir and ritonavir. Preferably, the combination is synergistic.

The term "combination" refers to a product comprising the defined nanoparticles and one or more of the compounds mentioned above potentially useful for treating respiratory syndrome-related coronaviruses, either in a single composition or in several compositions (or units), in which case the corresponding nanoparticles are distributed among the several compositions. Preferably, the combination refers to several compositions, in particular comprising one composition (or unit) per compound (compound as defined above) of the combination. The expression "one or more" when characterizing the combination refers to at least one, preferably 1, 2, 3, 4, or 5 compounds, more preferably, 1, 2 or 3 compounds, even more preferably 1 or 2 compounds.

When the combination is in the form of a single composition, the nanoparticles and the compounds present in the combination are always administered simultaneously.

When the combination is in the form of several compositions (or units), each of them having at least one of the components of the combination, the compositions or (units) may be administered simultaneously, sequentially or separately.

Simultaneous administration means that the nanoparticles, compounds or compositions (or units) are administered at the same time.

Sequential administration means that the nanoparticles, compounds or compositions (or units) are administered at different time points in a chronologically staggered manner.

Separate administration means that the nanoparticles, compounds or compositions (or units) are administered at different time points independently of each other.

In particular, the combinations for use according to the invention are administered as pharmaceutical compositions, which comprise the corresponding nanoparticles and (active) compounds and a pharmaceutically acceptable excipient, as previously defined.

The combinations for use according to the invention will typically be administered once or more times a day, for example 1, 2, 3 or 4 times daily, with typical total daily doses depending on the particular compound and severity of the disease, and may be easily determined by the skilled practitioner.

In this context, the present disclosure thus also provides a method of treatment or prevention of an infection by respiratory syndrome-related coronaviruses selected from the species *"Severe acute respiratory syndrome-related coronavirus"* and *"Middle East respiratory syndrome-related coronavirus"* comprising administering to a subject in need of treatment of said infection of an amount of coated iron oxide nanoparticles, the amount being effective to treat the infectious disease.

Further, in this context, the present disclosure provides the use of coated iron oxide nanoparticles for the preparation of a medicament for the treatment or prevention of an infection by respiratory syndrome-related coronaviruses selected from the species *"Severe acute respiratory syndrome-related coronavirus"* and *"Middle East respiratory syndrome-related coronavirus".*

The following examples represent specific embodiments of the present invention. They do not intend to limit in any way the scope of the invention defined in the present description.

## EXAMPLES

The following abbreviations have the meaning as follows:

IONPs-DMSA: Iron oxide nanoparticles coated with dimercaptosuccinic acid.

IONPs-APS: Iron oxide nanoparticles coated with (3-aminopropyl)triethoxysilane

DMSA-IONP-10: Iron oxide nanoparticles coated with dimercaptosuccinic acid having a particle size of 10 nm.

DMSA-IONP-16: Iron oxide nanoparticles coated with dimercaptosuccinic acid having a particle size of 16 nm.

APS-IONP-10: Iron oxide nanoparticles coated with (3-aminopropyl) triethoxysilane having a particle size of 10 nm.

## Characterization

The following methods were employed in the different analyses mentioned in the following examples.

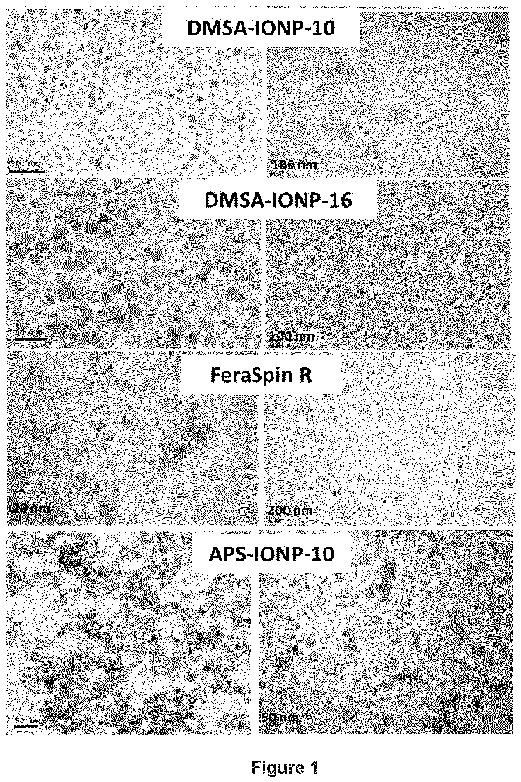

Particle size and shape were studied by transmission electron microscopy using a 100 keV JEOL microscope (see figure 1 for the TEM images of the nanoparticles of the invention). TEM samples were prepared by placing one drop of a dilute suspension of nanoparticles in water on a carbon coated copper grid and allowing the solvent to evaporate slowly at room temperature. The mean particle size and distribution were evaluated by measuring at least 250 particles. The $\sigma$ value in brackets in table 1 refers to the standard deviation from the mean values obtained by fitting the TEM data to a log normal distribution.

The phase of the iron oxide particles was identified by powder x-ray diffraction. The x-ray patterns were collected between 10° and 80° (2θ) in a Bruker D8 Advance diffractometer with Cu Kα radiation. The crystal size ($D_{XRD}$) was calculated from the broadening of the (311) reflection of the spinel structure following standard procedures.

The presence of adsorbed anions on the particle surface was also investigated by thermogravimetric (TG) analysis of the powders in a Seiko TG/ATD 320 U, SSC 5200. The analysis was performed at room temperature up to 900 °C at a heating rate of 10 °C $min^{-1}$ in an air flow.

Colloidal properties of the samples were studied in a Zetasizer Nano S, from Malvern Instruments. The hydrodynamic size of the particles in suspensions was measured by photon correlation spectroscopy and the electrophoretic mobility was measured as a function of pH at 25 °C, using $10^{-2}$M $KNO_3$ as electrolyte and $HNO_3$ and KOH to change the pH of the suspensions.

Magnetic characterization of the samples was carried out in a vibrating sample magnetometer (MLVSM9 MagLab 9 T, Oxford Instruments) at room temperature. Magnetization curves were recorded by first saturating the sample in a field of 5 T; then, the saturation magnetization (Ms), and the coercive field (Hc) were determined for each sample. Ms values were evaluated by extrapolating to infinite field the experimental results obtained in the high field range where magnetization linearly increases with 1/H. Samples were measured in powder form, pressed in a pellet.

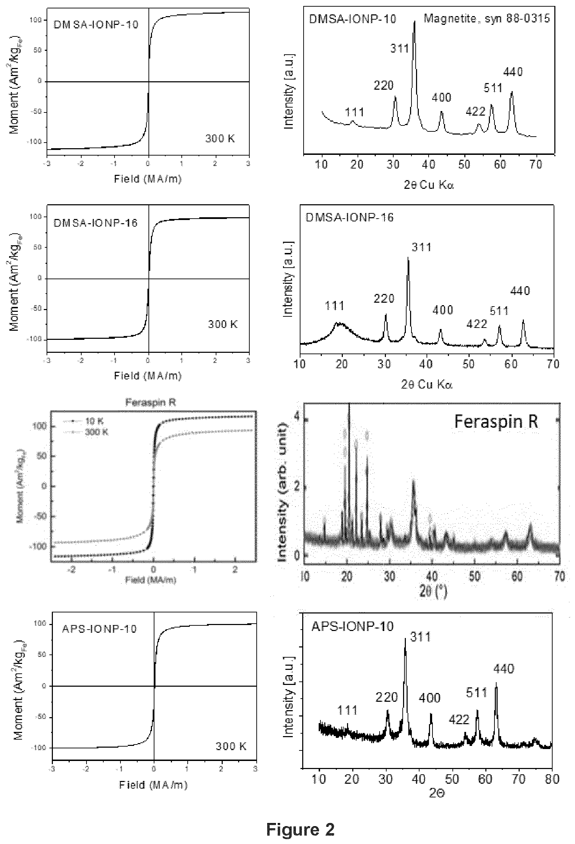

X-ray diffractograms of FeraSpin™ R (MRI contrast agent for pre-clinical imaging of nanoPET GmbH) and IONPs-DMSA samples showed peaks attributable to a spinel structure similar to magnetite. A typical X-ray diffraction pattern of FeraSpin™ R can be found in P. Bender et al., Nanotechnology 2018, 29, 425705 (see also figure 2). Crystal size calculated from the (311) peak broadening was in agreement with the size derived from TEM.

Electrophoretic mobility data were transformed to zeta potential values, which are related to the surface charge density and depend on the coating. Thus, the presence of coating such as carboxydextran or dimercaptosuccinic acid on the nanoparticle surface accounted for shifting the isoelectric point from pH 6-7, typical of magnetite, to lower pH values, leading a negative surface charge at neutral pH.

Cell lines, viruses and reagents

The African green monkey kidney-derived epithelial Vero E6 cells were kindly provided Centro Nacional de Biotecnología-CSIC. Vero E6 cells were grown in Dulbecco's modified Eagle's medium (DMEM) (Gibco, Invitrogen, CA) supplemented with 25 mM HEPES and 10% fetal bovine serum (Fisher).

SARS-CoV-2, isolated in Vero E6 cells, originating from a nasal swab from a patient infected in Madrid (Spain), was also kindly provided by Centro Nacional de Biotecnología-CSIC.

Example 1. Synthesis of coated nanoparticles

DMSA-IONPs suspensions. The iron oxide core was magnetite and the particle sizes of the coated nanoparticles were 10 and 16 nm as determined by TEM (denominated as DMSA-IONP-10 and DMSA-IONP-16, respectively). The coated nanoparticles were prepared by thermal decomposition of $Fe(acac)_3$ in the presence of oleic acid as surfactants and coated with dimercaptosuccinic acid by ligand exchange as described previously (A. Roca et al., J. Phys. Chem. B 2009, 113, 7033-7039; G. Salas et al., J. Mater. Chem. 22, 21065, 2012; J. Ovejero et al., Nanomaterials 2021, 11, 2059).

Magnetite nanoparticles were prepared by decomposition of iron (III) acetylacetonate 99% (Acros Organics, Geel, Belgium) in benzyl ether (99%; Acros Organics, Geel, Belgium) in the presence of oleic acid (OA; 80%; GPR Rectapur®, VWR, Leicestershire) and 1,2-dodecanediol 90 % (ODA; Sigma Aldrich, San Luis, MO, USA). The molar ratio $Fe(acac)_3$:OA:ODA was 1:3:2, and the concentration of the iron precursor was 0.1 M.

The mixture was overhead stirred at 100 rpm, and nitrogen (9.5 L/min) was flowed through the stirrer guide for 1.5 h (stirring and nitrogen flow were kept constant throughout the process). Then the reactor was heated up with an initial temperature ramp of 3 °C/min average, followed by a plateau at 200 °C for two hours, heating up again with a temperature ramp at a maximum power of 9 °C/min average, until the mixture was boiling and refluxing at 286 °C. Finally, the mixture was kept at the same temperature for 60 min to obtain 16 nm particles. Smaller particles of 10 nm were obtained introducing oleylamine (OAM) together with the oleic acid as surfactant in a molar ratio $Fe(acac)_3$:OA:OAM:ODA was 1:3:3:2.

Then, the stirring was stopped and the heating mantle removed in order to quench the reaction while maintaining the nitrogen flow. The product was washed three times with a mixture of toluene (99.5%; EssentQ®, Scharlau, Madrid, Spain) and ethanol (1:2 v/v), sonicated for 15 min, and magnetically separated.

Ligand exchange reaction of oleic acid for dimercaptosuccinic acid (DMSA) was used to transform hydrophobic magnetite nanoparticles into hydrophilic ones. First, particles were coagulated from the hydrophobic suspension (50 mg/5 mL) by addition of ethanol, centrifugation (2825 g, 10 min), and removal of the solution. Then, a mixture of 25 mL of toluene and a solution of 100 mg of DMSA in 5 mL of DMSO (dimethyl sulfoxide) was added to the coagulated particles, the obtained suspension was sonicated for 5 min, and mechanically stirred during 24 h. After that, toluene was added to the mixture reaction and the mixture was centrifuged again. Next, the supernatant containing the oleic acid coated particles was discarded. Finally, the precipitated nanoparticles coated with dimercaptosuccinic acid were successively mixed and centrifuged with ethanol and acetone several times to remove free oleic acid molecules. A final step consisted in dispersing the nanoparticles in alkaline water followed by a redispersion at pH 7. The dispersion was dialyzed, the pH was adjusted to 7, and a filtration through a 0.2 μm pore size syringe was performed.

FeraSpin™ R colloidal suspension was purchased from nanoPET-Pharma (Berlin, Germany). FeraSpin™ R is a colloidal suspension of clustered 5 nm superparamagnetic iron oxide - a mixture of magnetite and maghemite - nanoparticles that is synthesized by aqueous co-precipitation of Fe(II) and Fe(III) in the presence of carboxydextran as stabilizing agent. The hydrodynamic diameter is about 60 nm.

APS-IONP-10 suspensions. APS-coated nanoparticles were obtained by coprecipitation of a mixture of Fe(II)

and Fe(III) salts in aqueous based media, affording the iron oxide core, and then coating with (3-aminopropyl)triethoxysilane (APS) (Y. Luengo, Nanoscale 2013, 5, 11428-11437, DOI: 10.1039/c3nr02148c).

Typically, for the preparation of particles with a 10 nm diameter, a mixture of $FeCl_{3.6}H_2O$ (0.09 moles) and $FeCl_{2.4}H_2O$ (0.054 moles) in water (445 ml) were added to 75 ml of $NH_4OH$ (25%), under vigorous stirring. The precipitate was washed three times with distilled water by magnetic decantation. Then, particles were subjected to an acid treatment with 300 ml of $HNO_3$ (2 M) under stirring for 15 min. Then, nitric acid was removed by magnetic decantation, and 75 ml of $Fe(NO_3)_3$ (1 M in water) and 130 ml of water were added to the particles. The mixture was heated up to boiling temperature and stirred for 30 min. The particles were then cooled to room temperature and, by magnetic decantation, the supernatant was substituted by 300 ml of $HNO_3$ (2 M) and stirred for 15 min. Finally, the particles were washed three times with water and redispersed in distilled water. Nanoparticle surface coating with APS was performed by adding 1.22 ml (0.005 moles) of APS to a mixture of 10 ml of particles (28 g $Fe_2O_3$ per liter of distilled water) and 10 ml of methanol under strong stirring for 12 h. Methanol was eliminated by using a rotary evaporator. The APS-coated nanoparticle suspension was dialyzed, the pH was adjusted to 7 and the suspension filtered through a 0.2 $\mu$m pore size syringe.

All obtained nanoparticles were characterized by TEM, XRD, thermogravimetric analysis, photon correlation spectroscopy, vibrating-sample magnetometry and ICP-OES as described above. Table 1 summarizes the main structural and physiochemical features of the different IONPs.

Table 1. Characterization of the IONPs.

| Sample | Coating | Particle size TEM (nm) | Crystal size X-ray (nm) | Hydrodynami c size (nm) | Z-Potential (mV) | Ms (A $m^2/Kg_{Fe}$) |
|---|---|---|---|---|---|---|
| FeraSpin | Carboxydextr an | 4.8 ($\sigma$=0.27) | 5 | 66 (PDI= 0.24) | -36 | 93 |
| DMSA-IONP-10 | Dimercapto succinic acid | 9.9 ($\sigma$=0.22) | 10 | 65 (PDI= 0.25) | -33 | 119 |
| DMSA-IONP-16 | Dimercapto succinic acid | 16.8 ($\sigma$=0.16) | 13 | 74 (PDI= 0.23) | -27 | 99.4 |
| APS-IONP-10 | (3-aminopropyl)t riethoxysilane | 9 ($\sigma$=0.23) | 8 | 104.7 nm (PDI=0.26) | + 19 | 102 |
| PDI: polydispersity | | | | | | |

Example 2. Biological assays

Virus infections

To analyze the prophylactic effect of the nanoparticles, confluent monolayers of Vero E6 cells (24-well-format plates) were treated for 24 h with FeraSpin, DMSA-IONP-10, APS-IONP-10, and DMSA-IONP-16, at 50 and 250 $\mu$g/ml (approximately 1 mM and 5 mM, respectively) of iron. The studied concentrations were not found cytotoxic. The cells were then infected with SARS-CoV-2 (multiplicity of infection, MOI, 0.001), for 24 and 48 h. Cell culture media were collected and titrated 24 and 48 hours post infection (hpi). In addition, at 6 and 16 hpi, cells were collected and used for total RNA purification.

Alternatively, to analyze the therapeutic effect of the nanoparticles, confluent monolayers of Vero E6 cells (24-well-format plates) were infected with SARS-CoV-2 (MOI, 0.001), and the nanoparticles were added to the media at 1 hpi, using the same concentrations as those described for the prophylactic effect. Extracellular media were collected and titrated 24 and 48 hpi. In addition, at 6 and 16 hpi, cells were collected and used for total RNA purification.

Virus titrations

SARS-CoV-2 virus titrations were performed in Vero E6 cells grown in 24-well plates and infected with 10-fold serial dilutions of the cell culture media containing the extracellular virus. After 1h absorption, cells were overlaid with low electroendosmosis agarose (Pronadisa) and incubated for 3 days at 37°C. For all virus titrations, cells were fixed with 10% formaldehyde in phosphate buffer saline (PBS) and permeabilized with 20% methanol. Viral plaques were visualized and counted using crystal violet.

Analysis of virus replication and transcription

Total RNA from untreated or IONPs-treated cells either mock-infected or SARS-CoV-2-infected were extracted using a total RNA extraction kit (Omega Bio-tek, GA, USA) following the manufacturer's recommendations. Purified RNA (1 μg) was reverse-transcribed to cDNA using a high-capacity cDNA reverse-transcription kit (Applied Biosystems) and random primers.

To analyze the effect of the treatments with nanoparticles on virus replication, qPCRs using the primers SARS-2-RdRp-15431-VS (5'GTGAAATGGTCATGTGTGGCGG-3') and SARS-2-RdRp-15530-RS (5'-CAAATGTTAAAAACACTATTAGCATA-3'), complementary to the viral genomic RNA (gRNA) were performed according to the procedure in T. Toptan et al., Int. J. Mol. Sci. 2020, 21(12), 4396).

To analyze the effect of the nanoparticles on virus transcription, the primers, SARS-2-leader-VS: 5'-TCCCAGGTAACAAACCAACCAACT, complementary to the leader sequence; and SARS-2-7a-RS: 5'-AAATGGTGAATTGCCCTCGT-3, complementary to gene 7 open reading frame, were used, to amplify the gene 7 subgenomic messenger RNA (sg mRNA) by qPCR according to the procedure in Alexandersen et al., Nature Communications, 2020, 6059. In all the cases, GAPDH was used to normalize the data using the 5'-TGCCATGGGTGGAATCATATTGGA-3' (sense) and 5'-TCGGAGTCAACGGATTTGGGTCGT-3 (antisense) primers. Quantification was achieved using the threshold cycle (2$^{-\Delta\Delta CT}$) method (K. J. Livak et al, Methods, 2001, 25(4), 402-408).

Analysis of the contribution of oxidative stress to the antiviral effect of the IONPs

Confluent monolayers of Vero E6 cells (24-well-format plates) were treated during 24 h with N-acetylcysteine (NAC), recognized as a reactive oxygen species scavenger, at a 200 μM concentration or left untreated, as control. Then, the cells were infected with SARS-CoV-2 (MOI, 0.001), and at 1 hpi, the extracellular medium containing the virus was replaced with a suspension of the IONPs and NAC solution (200 μM) or with a suspension of the IONPs without NAC as control experiment. Media were collected and titrated at 48 hpi.

Effect of nanoparticles on viral production

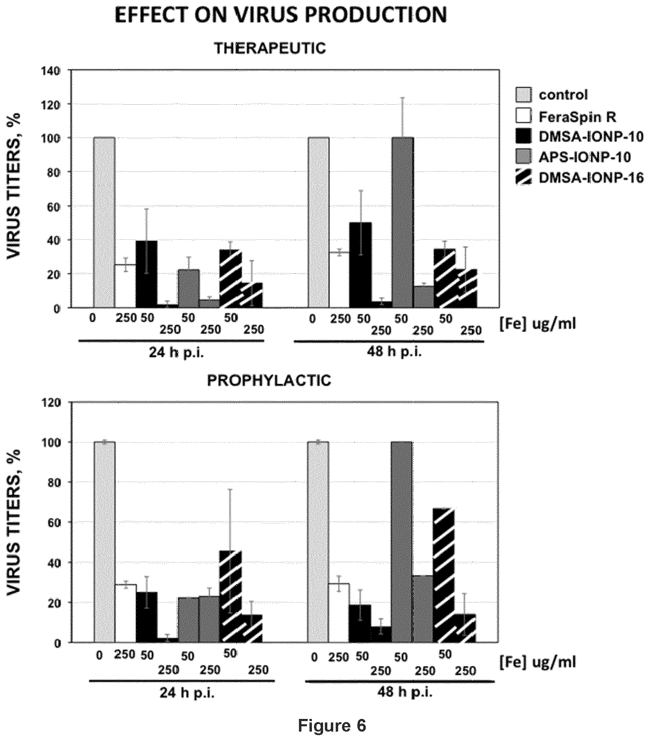

In order to verify whether iron, in the form of iron oxide nanoparticles (FeraSpin, DMSA-IONP-10, APS-IONP-10, and DMSA-IONP-16), affect SARS-CoV-2 production, cells were either treated with the nanoparticles 24 h before infection (prophylactic effect), or 1 h after infection (therapeutic effect), and virus titers at different times post-infection were measured (see "Virus titrations" section above). Interestingly, for both the therapeutic and prophylactic effect, a dose-dependent effect of nanoparticles on reducing infectious viruses produced by infected cells was observed (see Figure 6).

For the therapeutic treatment, the highest, non-cytotoxic concentrations of all the IONPs decreased SARS-CoV-2 production at 24 and 48 hpi, being DMSA-IONP-10 and APS-IONP-10 the nanoparticles reducing viral titers to a greater extent. In fact, the highest concentrations (250 μg Fe/ml that is about 5mM) of DMSA-IONP-10 and APS-IONP-10 reduced virus titers by 98, and 95.5%, respectively, at 24 hpi, and by 96.25, and 87.5 %, respectively, at 48 hpi.

For the prophylactic treatment, the highest concentrations of IONPs reduced virus titers at 24 and 48 hpi. The maximum reduction of virus titers in cells which were first treated with the nanoparticles and then infected was observed using the highest concentration of DMSA-IONP-10, which reduced viral titers by 98% and 90% at 24 and 48 hpi, respectively. These results indicated that the treatment of cells with NPs reduced the production of SARS-CoV-2 infectious viruses.

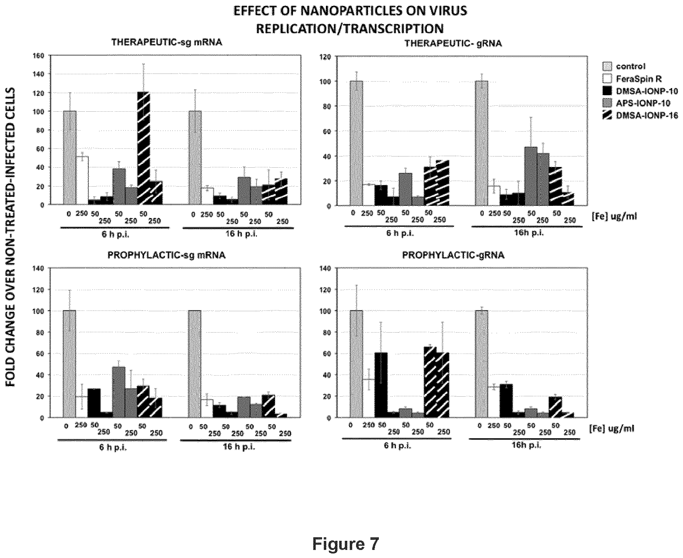

In order to gain insight on whether the treatment of cells with IONPs directly affects virus replication and/or transcription, cells were treated with IONPs before or after SARS-CoV-2 infection and the amounts of genomic RNA (gRNA), which determines virus replication, and sg mRNA, which determines virus transcription, were measured by qRT-PCR at 6 and 16 hpi. Remarkably, the treatment of cells before or after SARS-CoV-2 infection with the highest doses of IONPs decreased the amounts of gRNA and gene 7 sg mRNA at 6 and 16 hpi in both therapeutic and prophylactic treatments (see figure 7). These results strongly suggested that the treatment of cells with IONPs impaired virus replication and transcription.

It has been shown that the IONPs induce oxidative stress (Mulens-Arias et al., Biomaterials, 2015, 52:494-506; Khan et al., Biomaterials, 2012, 33(5):1477-88; Portilla et al. ACS Appl. Mater. Interfaces, 2021, 13(7):7924-7944). To analyze whether the oxidative stress induced by the IONPs is responsible for their antiviral effect, cells were treated with the IONPs and NAC, a ROS scavenger, and viral titers were measured at 48 hpi. Interestingly, in control cells, non-treated with the IONPs, viral titers were similar in cells treated with NAC and in cells where NAC was not applied. However, in cells treated with DMSA-IONP-10 and DMSA-IONP-10, viral titers increased when the cells were additionally treated with NAC, compared to cells incubated in absence of NAC. This effect was not observed in the cells treated with FeraSpin™ R and APS-IONP-10 (data not shown). These results suggested that the induction of oxidative stress by the IONPs DMSA-IONP-10 and DMSA-IONP-16 was at least partially responsible for the antiviral effect observed for these IONPs.

Cytotoxicity assays by PrestoBlue

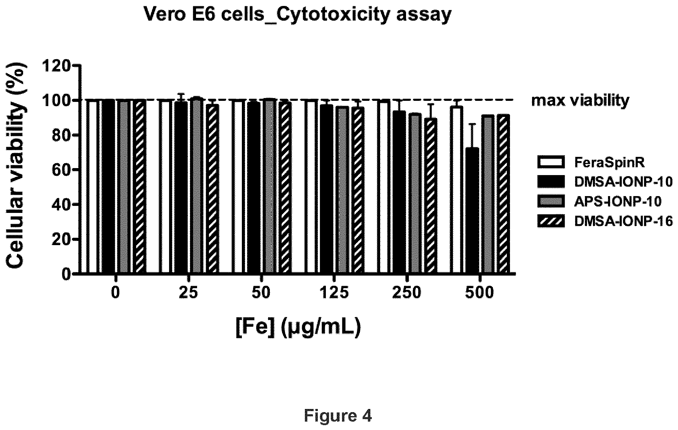

Cell viability was determined with the colorimetric Presto Blue assay (Invitrogen). Presto Blue reagent contains a cell permeable blue and non-fluorescent solution of resazurin, which can be metabolized inside cells to a red and fluorescent compound called resorufin. The change of color and fluorescence serves as a cell viability indicator. Vero E6 cells were seeded in a 96-well plate at a density of $3 \times 10^4$ cells in a total volume of $100 \mu l$ per well. After 24h incubation at 37°C, Vero E6 cells were incubated with different concentrations of nanoparticles suspensions (0-500 $\mu$g Fe/ml) for 24h. After nanoparticle incubation (FeraSpinR, DMSA-IONP-10, DMSA-IONP-16 and APS-IONP-10, PrestoBlue reagent was added to each well, incubated in the same culture conditions for 2h and fluorescence was measured (560nm excitation; 590nm emission). Cell viability (see Figure 4) is indicated as percentage of fluorescence of treated cells compared to fluorescence of the non-treated cells following the equation:

$$Cell\ Viability\ (\%) = \frac{fluorescence\ treated\ cells - medium\ fluorescence}{fluorescence\ non\ treated\ cells - medium\ fluorescence}\ x\ 100$$

As it appears in figure 4, the coated iron oxide nanoparticles are not cytotoxic up to a concentration of 250 $\mu$g Fe/mL (about 5 mM) in Vero E6 cell lines (cell viability > 90%). At 500 $\mu$g Fe/mL concentration (about 9 mM), the IONPs-DMSA begin to show negligible cytotoxicity (cell viability 70-90%) in Vero E6 cells.

Quantification of iron uptake by ICP-OES

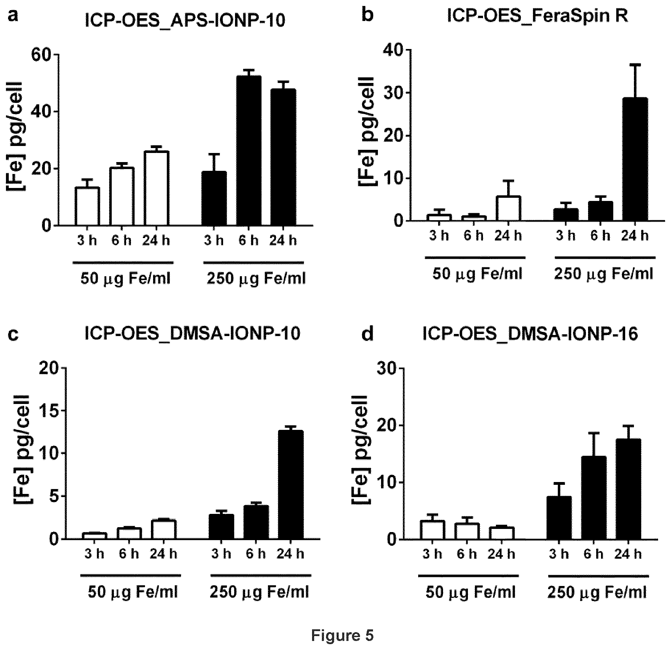

Vero E6 cells were seeded in a 6-well plate at a density of $1 \times 10^5$ cells per well and cultured for 24 h at 37 °C. After this, the Vero E6 cells were incubated with different nanoparticles at two concentrations: FeraSpin R (50 or 250 $\mu$g Fe/mL, that is about 1 or 5 mM Fe), DMSA-IONP-10 (50 or 250 $\mu$g Fe/mL, that is about 1 or 5 mM Fe), APS-IONP-10 (50 or 250 $\mu$g Fe/mL, that is about 1 or 5 mM Fe) and DMSA-IONP-16 (50 or 250 $\mu$g Fe/mL, that is about 1 or 5 mM Fe) were then added and incubated in the same culture conditions for 3, 6 or 24 h. Subsequently, the cells were then washed three times with PBS to remove non-internalized nanoparticles, harvested and counted in a Neubauer chamber. The samples were digested in $HNO_3$ (1 mL) and $H_2O_2$ (1 mL) for 1h at 90 °C, and the amount of IONPs internalized by Vero E6 cells after incubation with different doses of IONPs at different incubation times (see Figure 5) was determined by ICP-OES (Inductively coupled plasma - optical emission spectrometry) with a Perkin Elmer-2400 instrument.

FeraSpin R and APS-IONP-10 were internalized to a higher degree in Vero E6 cells at all concentrations, highlighting the potential impact of their physical and chemical properties on the iron uptake and therapeutic/prophylactic effects into target cells.

**Claims**

1. A coated iron oxide nanoparticle for use in the treatment or prevention of an infection caused by respiratory syndrome-related coronaviruses selected from the species Severe acute respiratory syndrome-related coronavirus and Middle East respiratory syndrome-related coronavirus.

2. The coated iron oxide nanoparticle for use according to claim 1, wherein the respiratory syndrome-related coronavirus is selected from the group consisting of SARS-CoV-2, SARS-CoV and MERS-CoV, preferably SARS-CoV-2.

3. The coated iron oxide nanoparticle for use according to claims 1 and 2, wherein the size of the coated nanoparticle is comprised between 2 and 50 nm, preferably between 4 and 20 nm.

4. The coated iron oxide nanoparticle for use according to claims 1 to 3, wherein the nanoparticle is coated with inorganic materials selected from silica, carbon, non-metal elements, metal oxides and metal sulphides; or with a neutrally charged organic molecule selected from the group consisting of biocompatible organic molecules, surfactants, polymers and biomacromolecules.

5. The coated iron oxide nanoparticle for use according to claims 1 to 3, wherein the nanoparticle is coated with a neutrally charged organic molecule containing hydroxyl, amino and/or thiol groups.

6. The coated iron oxide nanoparticle for use according to claim 5, wherein the neutrally charged organic molecule used for coating the nanoparticle has a molecular weight between 80 and 100000 Dalton, preferably between 80 and 50000 Dalton.

7. The coated iron oxide nanoparticle for use according to claims 1 to 6, wherein the nanoparticle is coated with dextran, carboxydextran, carboxymethyldextran, starch, PEGylated starch, (3-aminopropyl)triethoxysilane or dimercaptosuccinic acid (DMSA).

8. The coated iron oxide nanoparticle for use according to claims 1 to 7, wherein the size of the coated iron nanoparticle is between 5 and 20 nm, the iron oxide nanoparticles being covered by carboxydextran and having a hydrodynamic size comprised between 50 and 80 nm.

9. The coated iron oxide nanoparticle for use according to claims 1 to 7, wherein the coated nanoparticle is selected from the group consisting of FeraSpin™ R, Magtrace®, NanoTherm® AS1, Endorem®, Sinerem®, Ferumoxytol®, Resovist® and Clariscan®.

10. The coated iron oxide nanoparticle for use according to claims 1 to 7, wherein the size of the coated iron nanoparticle is comprised between 5 and 20 nm, the iron oxide nanoparticles being covered by dimercaptosuccinic acid and having a hydrodynamic size comprised between 50 and 80 nm.

11. The coated iron oxide nanoparticle for use according to claims 1 to 7, wherein the size of the coated iron nanoparticle is comprised between 5 and 15 nm, preferably is about 9 nm, the iron oxide nanoparticles being covered by (3-aminopropyl)triethoxysilane and having a hydrodynamic size comprised between 60 and 125 nm.

12. The coated iron oxide nanoparticle for use according to claims 1 to 11, alone or in combination with a pharmaceutical carrier, for oral, intranasal or parenteral administration.

13. The coated iron oxide nanoparticle for use according to claim 12 wherein the parenteral administration is performed by intravenous (i.v), intracoronary (i.c), intramuscular (i.m), intraperitoneal (i.p), or subcutaneous (s.c.) injection.

14. The coated iron oxide nanoparticle for use according to claim 12 wherein the intranasal administration is performed by aerosols.

Figure 1

**Figure 2**

Figure 3

Figure 4

Figure 5

# EFFECT ON VIRUS PRODUCTION

Figure 6

Figure 7

## EUROPEAN SEARCH REPORT

**Application Number**

EP 21 38 2973

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Martins Elisama S. ET AL: "Potential Use of DMSA-Containing Iron Oxide Nanoparticles as Magnetic Vehicles against the COVID-19 Disease", Materials Science inc. Nanomaterials & Polymers, 17 August 2021 (2021-08-17), pages 7931-7935, XP55908441, Retrieved from the Internet: URL:https://www.ncbi.nlm.nih.gov/pmc/articles/PMC8441750/pdf/SLCT-6-7931.pdf [retrieved on 2022-04-04] | 1-7,10 | INV. A61K9/51 |
| Y | * the whole document * | 8,9, 11-14 | |
| | ----- | | |
| Y | Abo-Zeid Yasmin ET AL: "A molecular docking study repurposes FDA approved iron oxide nanoparticles to treat and control COVID-19 infection", Eur J Pharm Sci. 153:105465., 12 July 2020 (2020-07-12), XP055854500, DOI: 10.1016/j.ejps.2020.105465 Retrieved from the Internet: URL:https://www.ncbi.nlm.nih.gov/pmc/articles/PMC7354764/pdf/main.pdf [retrieved on 2021-10-25] * the whole document * | 8,9, 11-14 | |
| | ----- | | TECHNICAL FIELDS SEARCHED (IPC) |
| | -/-- | | A61K |

The present search report has been drawn up for all claims

| Place of search | Date of completion of the search | Examiner |
|---|---|---|
| Munich | 25 April 2022 | Raposo, Antonio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 21 38 2973

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BOBO DANIEL ET AL: "Nanoparticle-Based Medicines: A Review of FDA-Approved Materials and Clinical Trials to Date", PHARMACEUTICAL RESEARCH, SPRINGER US, NEW YORK, vol. 33, no. 10, 14 June 2016 (2016-06-14) , pages 2373-2387, XP036045079, ISSN: 0724-8741, DOI: 10.1007/S11095-016-1958-5 [retrieved on 2016-06-14] * abstract * * page 2382, left-hand column, paragraph 2 * | 8,9, 11-14 | |
| Y | ARAMI HAMED ET AL: "In vivo delivery, pharmacokinetics, biodistribution and toxicity of iron oxide nanoparticles", CHEMICAL SOCIETY REVIEWS, vol. 44, no. 23, 1 January 2015 (2015-01-01), pages 8576-8607, XP055908754, UK ISSN: 0306-0012, DOI: 10.1039/C5CS00541H Retrieved from the Internet: URL:https://www.ncbi.nlm.nih.gov/pmc/artic les/PMC4648695/pdf/nihms-724691.pdf> * 2. IONPs pharmacokinetics; page 3 * | 12-14 | TECHNICAL FIELDS SEARCHED (IPC) |

The present search report has been drawn up for all claims

| Place of search | Date of completion of the search | Examiner |
|---|---|---|
| Munich | 25 April 2022 | Raposo, Antonio |

EPO FORM 1503 03.82 (P04C01)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. J. GUZIK et al.** *Cardiovasc Res,* 2020, vol. 116 (10), 1666-1687
- **C. LADECOLA et al.** *Cell,* 2020, vol. 183 (1), 16-27
- **PORTILLA et al.** *ACS Appl. Mater. Interfaces,* 2021, vol. 13 (7), 7924-7944
- **KUMAR et al.** *J. Infect. Chemother.,* 2019, vol. 25 (5), 325-329
- **KIM et al.** *Small,* 2017, vol. 13 (32), 1700818
- **WANG et al.** *Cell Discovery,* 2018, vol. 4, 14
- **Y. ABO-ZEID et al.** *Eur. J. Pharm. Sci.,* 2020, vol. 153, 105465
- **Y. PAN.** *Small,* 2007, vol. 3, 1941-1949
- **ZHAO et al.** *Open Forum Infectious Diseases,* 2020, vol. 7 (7), ofaa250
- **SONNWEBER et al.** *Respiratory Research,* 2020, vol. 21 (1), 276
- **P. BENDER et al.** *Nanotechnology,* 2018, vol. 29, 425705
- **A. ROCA et al.** *J. Phys. Chem. B,* 2009, vol. 113, 7033-7039
- **G. SALAS et al.** *J. Mater. Chem.,* 2012, vol. 22, 21065
- **J. OVEJERO et al.** *Nanomaterials,* 2021, vol. 11, 2059
- **Y. LUENGO.** *Nanoscale,* 2013, vol. 5, 11428-11437
- **T. TOPTAN et al.** *Int. J. Mol. Sci.,* 2020, vol. 21 (12), 4396
- **ALEXANDERSEN et al.** *Nature Communications,* 2020, 6059
- **K. J. LIVAK et al.** *Methods,* 2001, vol. 25 (4), 402-408
- **MULENS-ARIAS et al.** *Biomaterials,* 2015, vol. 52, 494-506
- **KHAN et al.** *Biomaterials,* 2012, vol. 33 (5), 1477-88